# EUROPEAN PATENT SPECIFICATION

(11) **EP 0 919 549 B1**
(45) Date of publication and mention of the grant of the patent: **11.12.2002**
(21) Application number: 98123055.0
(22) Date of filing: 04.04.1997
(51) Int. Cl.: C07D 249/08, C07D 487/04, G03C 7/38

(54) **3-(4-Tert-butylphenyl)-1H-1,2,4-trizaol-5-yl acetic acid and uses thereof, particularly for the preparation of photographic couplers**
3-(4-Tert-butylphenyl)-1H-1,2,4-triazol-5-ylessigsäure und ihre Verwendungen, insbesondere zur Herstellung von fotographischen Kupplern
Acide 3-(4-tert-butylphényl)-1H-1,2,4-triazol-5-yl acétique et ses utilisations, en particulier dans la préparation de coupleurs photographiques

(30) Priority: 04.04.1996 JP 8268896
(43) Date of publication of application: 02.06.1999
(62) Divisional of application: 97105609.8
(73) Proprietor: FUJI PHOTO FILM CO., LTD., Kanagawa 250-01 (JP)
(72) Inventor: Shimura, Yoshio, Minami-Ashigara-shi, Kanagawa-ken (JP); Shimada, Yasuhiro, Minami-Ashigara-shi, Kanagawa-ken (JP)
(74) Representative: HOFFMANN - EITLE

(56) References cited:
- EP-A- 0 714 892
- EP-A- 0 720 981
- JP-A- 7 048 376
- US-A- 5 256 526

## Description

### FIELD OF THE INVENTION

The present invention relates to an 1H-1,2,4-triazol-5-yl acetic acid compound that is useful as a coupler intermediate for photography.

### BACKGROUND OF THE INVENTION

With respect to the method for synthesizing 1H-1,2,4-triazol-5-yl acetic acids, synthetic examples can be found, for example, in IZV, Akad, Nauk, SSSR, Ser. Khim., 9,2075 (1990); Chem Pharm. Bull., 36.96 (1988), and JP-A ("JP-A" means unexamined published Japanese patent application) No. 172357/1994. However, because in the methods described in these publications, for example, the yield is low, the reaction procedure and post-treatment are complicated, and the reaction takes much time, it is difficult to obtain the particular compounds efficiently in a high yield, which means that the methods are unsatisfactory as industrial production methods. As a technique relatively similar to the present invention, a method is disclosed in JP-A No. 48376/1995. Although the method disclosed therein is improved in comparison with the above synthetic examples, however, for example, the method must use, as a raw material, a compound represented by formula (2) given below in a free form (in the formula, n = 0), and in Step 2, an aqueous NaOH solution as a base is used, in which the method is different from the present invention. Further, in the method disclosed therein the solvent is removed by distillation, making the procedure complicated, and the yield is low, which means that the method is unsatisfactory as an industrial production method. Therefore, there is strong need for the development of a method for synthesizing 1H-1,2,4-triazol-5-yl acetic acids in a high yield, wherein the reaction procedure and post-treatment are simple.

### SUMMARY OF THE INVENTION

An object of the present invention is to provide a 1H-1,2,4-triazol-5-yl acetic acid compound that is useful as a coupler intermediate for photography.

Other and further objects, features and advantages of the invention will appear more fully from the following description.

### DETAILED DESCRIPTION OF THE INVENTION

The inventors of the present invention have studied intensively to attain the above objects and, as a result, have completed the present invention. That is, the present invention provides: 3-(4-tert-butylphenyl)-1H-1,2,4-triazol-5-yl acetic acid.

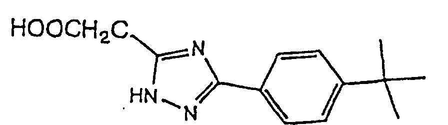

Among the 1H-1,2,4-triazol-5-yl acetic acid compounds, 3-(4-tert-butylphenyl)-1H-1,2,4-triazol-5-yl acetic acid is a novel compound that has not yet appeared in the literature, and it is useful as a raw material for the synthesis of pyrrolotriazole cyan couplers that are excellent in coupling activity, heat stability, and light/heat fastness and hue of the dye image, which couplers are described in U.S. Patent Nos. 5,256,526 and 5,384,236. Further, it is advantageous that the raw material compound: p-tert-butylphenylhydrazide, corresponding to formula (1) below, which is a raw material for the synthesis of 3-(4-tert-butylphenyl)-1H-1,2,4-triazol-5-yl acetic acid, can be obtained very inexpensively through simple procedures in a high yield from 4-tert-butylbenzoic acid vinyl ester, which is known as a modifier for synthetic resins.

A method for preparing a 1H-1,2,4-triazol-5-yl acetic acid compound comprises reacting a compound represented by the below-given formula (1), wherein R1 represents a alkyl group or aryl group (which alkyl or aryl group may be substituted one in this invention), with a compound represented by the below-given formula (2), wherein R2 represents an alkyl group, R3 represents an alkyl group or aryl group, A represents an acid substance selected from the group consisting of HCl, HBr, HI, phosphoric acid, H₂SO₄, and p-toluenesulfonic acid, and n is 0, 0.5 or 1, in the presence of water, a water-soluble organic solvent, and at least one of NaOH and CH₃COOM, in which latter M represents an alkalimetal (e.g. Na, K, Li), to produce a compound represented by the below-given formula (3), wherein R1 and R2 have the same meanings as those in formulas (1) and (2); and then adding M₂CO₃, wherein M has the same meaning as defined above, followed by heating, thereby obtaining a compound represented by the below-given formula (4), wherein R1 has the same meaning as defined in formulas (1) and (3):

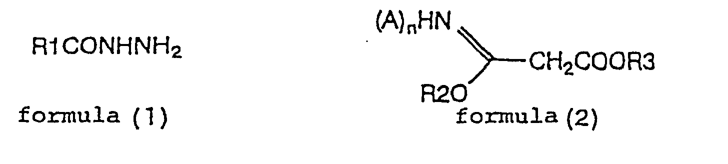

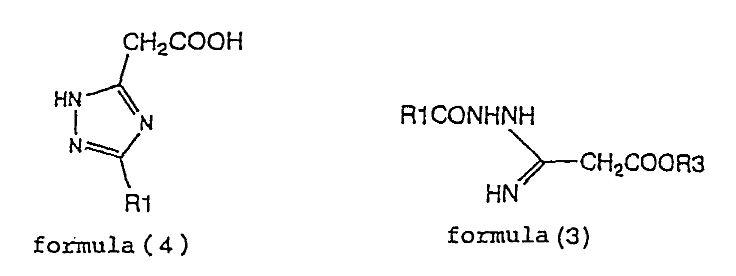

A method for preparing a 1H-1,2,4-triazol-5-yl acetic acid compound comprises heating a compound represented by formula (3) in the presence of water and M₂CO₃, wherein M has the same meaning as defined in the above (1), to obtain a compound represented by formula (4).

Next, the compounds represented by formulas (1), (2), and (3), that is, the raw materials, and the compound represented by formula (4), that is, the intended compound, are described in detail. In formulas (1), (3), and (4), R1 is an optionally substituted monocyclic or condensed-ring aryl group that preferably has a total number of carbon (hereinafter abbreviated to carbon number) of 6 to 36, or it is an optionally substituted straight-chain, branched-chain, or cyclic alkyl group that preferably has a carbon number of 1 to 30, and examples of the substituents include a cyano group, a halogen atom (Cl, Br, F, I), an alkyl group, an alkoxy group, a carbonamido group, a sulfonamido group, an alkoxycarbonyl group, an aryloxycarbonyl group, an acyloxy group, and a nitro group.

In formulas (1), (3), and (4), more preferably R1 represents an optionally substituted aryl group having a carbon number of 6 to 30 (e.g. phenyl, 1-naphthyl, p-tolyl, o-tolyl, p-tert-butylphenyl, o-2-ethylhexyloxyphenyl, 4-methoxyphenyl, 4-methyl-3-nitrophenyl, 3,5-di-chlorophenyl, p-cyanophenyl, 2,4-di-tert-pentylphenyl, pentafluorophenyl, and p-methanesulfoneamidophenyl), or it is an optionally substituted alkyl group having a carbon number of 1 to 24 (e.g. methyl, isopropyl, tert-butyl, cyclohexyl, 2-ethylhexyl, 1,1,3,3-tetramethylbutyl, dodecyl, vinylmethyl, trifluoromethyl, methoxyethyl, ethoxycarbonylmethyl, and phenoxyethyl).

Particularly preferably, R1 represents an aryl group having a carbon number of 6 to 24 (e.g. phenyl, o-tolyl, p-tert-butylphenyl, 4-methyl-3-nitrophenyl, 3,5-di-chlorophenyl, and p-cyanophenyl), or an alkyl group having a carbon number of 1 to 20 (e.g. methyl, isopropyl, t-butyl, cyclohexyl, and phenoxyethyl), and most preferably it represents a 4-methyl-3-nitrophenyl group, a p-tert-butylphenyl, a tert-butyl group, a cyclohexyl group, or a phenoxyethyl group.

When R2 in formula (2) and R3 in formulas (2) and (3) are alkyl groups, the alkyl groups are independently alkyl groups that preferably have a carbon number of 1 to 10, and more preferably 1 to 4 (e.g. methyl, ethyl, and propyl). When R3s in formulas (2) and (3) are aryl groups, the aryl groups are independently aryl groups having a carbon number of 6 to 20, and more preferably 6 to 12 (e.g. phenyl, p-tolyl, and o-tolyl). Preferably R2 in formula (2) and R3 in formulas (2) and (3) are alkyl groups, and methyl and ethyl groups are particularly preferable.

The reaction can be represented by the following scheme:

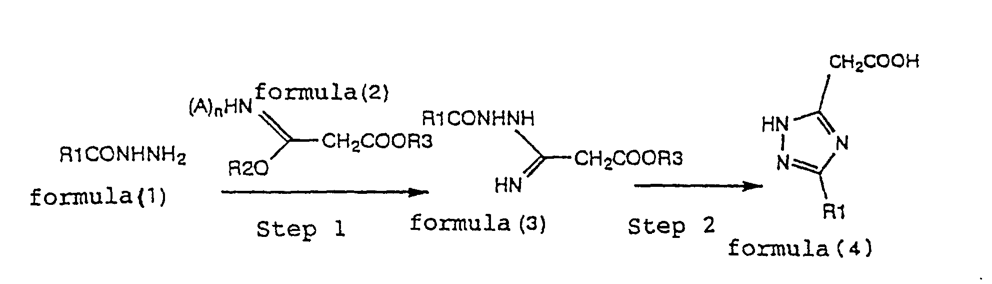

In Step 2, the ester group (-COOR3) in the compound of formula (3) is hydrolyzed upon a reaction in basic condition, to form an intermediate carboxylic acid. Then, the carboxylic acid formed from the compound of formula (3) is subjected to a ring-closure reaction, to form the compound of formula (4).

Now, how to carry out continuously Steps 1 and 2 stated in the above (1) is described in detail. The base used in Step 1 is NaOH or CH₃COOM, in which latter M represents an alkali metal, and examples of CH₃COOM are CH₃COOLi, CH₃COONa, and CH₃COOK, with particular preference given to CH₃COONa. Further, the amount of the base to be used (in a molar ratio to the compound represented by formula (1)) is from 0.8 to 10 : 1, preferably from 0.8 to 3.0 : 1, and particularly preferably from 1.0 to 1.5 : 1.

As the solvent used in Step 1, water and a water-soluble organic solvent (e.g. acetonitrile, tetrahydrofuran (THF), dioxane, MeOH, EtOH, isopropyl alcohol, acetone, and N,N-dimethylacetamide) can be mentioned, with preference given to acetonitrile and THF as the organic solvent, and particularly to acetonitrile. The amount of solvent (water + organic solvent) for 1 mol of the compound represented by formula (1) is 0.5 to 3.0 liters, and particularly preferably 0.5 to 1.5 liters. The amount of the compound represented by formula (2) (in a molar ratio to the compound represented by formula (1)) is from 0.8 to 1.5 : 1, preferably from 0.9 to 1.2 : 1, and particularly preferably from 1.0 to 1.1 : 1.

The reaction temperature is -30 to 80 °C, preferably -10 to 60 °C, and particularly preferably 0 to 30 °C. The reaction time is 1 min to 24 hours, preferably 5 min to 6 hours, and more preferably 5 min to 3 hours.

Next, Step 2 is described in detail. Examples of M₂CO₃ (wherein M represents an alkali metal), which is added to the reaction liquid containing the compound represented by formula (3) produced in Step 1, include Li₂CO₃, Na₂CO₃, and K₂CO₃, with preference given to Na₂CO₃ and K₂CO₃, and particularly to Na₂CO₃. The amount of M₂CO₃ (in a molar ratio to the raw material compound represented by formula (1) used in Step 1) is from 1 to 10 : 1, preferably from 1.5 to 5 : 1, and more preferably from 2 to 3 : 1. The amount of water added in Step 2 is 1 to 2 liters, and preferably about 1 liter, to 1 mol of the raw material compound represented by formula (1). The reaction temperature is 0 to 80 °C, preferably room temperature (20 °C) to 80 °C, and more preferably 65 to 80 °C. The reaction time is 30 min to 30 hours, and preferably 30 min to 2 hours.

The reaction in Step 2, wherein the compound represented by formula (3) stated under the above embodiment of (2) is used as a raw material compound, can be carried out under the same reaction conditions as in Step 2 stated under the above embodiment of (1). That is, the type of M₂CO₃ (wherein M represents an alkali metal) used in Step 2 is the same as that in the above (1), and the amount thereof (in a molar ratio to the raw material compound represented by formula (3)) is from 1 to 10 : 1, and preferably from 2 to 3 : 1. The amount of water to be added is 1 to 3 liters, and preferably about 2 liters, to 1 mol of the raw material compound represented by formula (3). The reaction temperature is 0 to 80 °C, preferably 20 to 80 °C, and more preferably 65 to 80 °C. The reaction time is 30 min to 3 hours, and preferably 30 min to 2 hours.

According to the present invention, 1H-1,2,4-triazol-5-yl acetic acid compounds that are useful as intermediates for photographic couplers can be obtained simply in a high yield.

The 3-(4-tert-butylphenyl)-1H-1,2,4-triazol-5-yl acetic acid compound of the present invention gives a coupler which can form by a coupling reaction with an oxide of a color developing agent a dye that excels in hue and fastness, other dyes formed from a coupler obtained from a 3-phenyl-1H-1,2,4-triazol-5-yl acetic acid compound. Further, it is advantageous that the 3-(4-tert-butylphenyl)-1H-1,2,4-triazol-5-yl acetic acid compound can be synthesized at lower cost since a starting material, a compound of formula (1) (R1: p-tert-butylphenyl) can be synthesized from a p-tert-butylbenzoic acid vinyl ester that is easily available as a polymer modifying agent.

### EXAMPLES

Now, the present invention is described with reference to examples in detail.

### Example 1

A mixture of 51.4 g of sodium acetate, 150 ml of water, and 500 ml of acetonitrile was stirred at room temperature, and 100.7 g of 3-methoxy-3-iminoethylpropionate hydrochloride was added thereinto, followed by stirring for 5 min. Then, 96 g of 4-tert-butylbenzoic acid hydrazide was added thereto, followed by stirring at 15 °C for 2 hours. Then 850 ml of water and 106 g of sodium carbonate were added to the reaction system, and the reaction mixture was heated on a water bath under reflux. After 2 hours, the inside temperature was cooled with water to 30 °C, and 170 ml of concentrated hydrochloric acid was added, dropwise, to neutralize the reaction mixture. After the neutralization, the reaction liquid was cooled with ice, and the deposited crystals of 3-(4-tert-butylphenyl)-1H-1,2,4-triazol-5-yl acetic acid were filtered.

Obtained amount: 103.7 g; yield: 80%; melting point: 118 to 120 (decomposed).

¹H-NMR (CDCl₃) δ/ppm: 1.3 ppm (S. 9H), 4.18 (S. 2H), 7.4 to 7.55 (d. 2H), 7.8 to 8.0 (d. 2H)

### Example 2

A mixture of 26 g of 3-[(4-tert-butylbenzoyl)-hydrazino]-3-iminopropionic acid ethyl ester, 18.1 g of sodium carbonate, and 170 ml of water was heated and stirred, with the internal temperature being 80 °C. After 2 hours, the reaction liquid was cooled, and 29 ml of HCl was added, dropwise, to neutralize the reaction liquid, with the internal temperature being 30 °C. The deposited crystals were filtered, to obtain crystals of 3-(4-tert-butylphenyl)-1H-1,2,4-triazol-5-yl acetic acid. Obtained amount: 21 g; yield: 95%.

Specific examples of the compound represented by formula 4 that is obtained in the above Steps 1 and 2 are shown below. 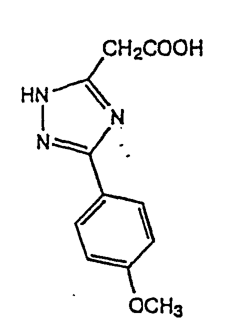 Melting point: 93 to 95 °C (decomposed) 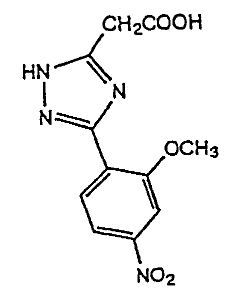 Melting point: 222 °C 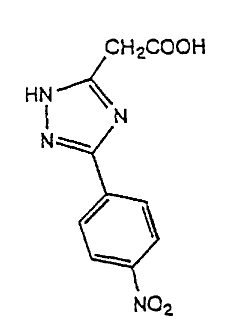 Melting point: 234 to 235 °C 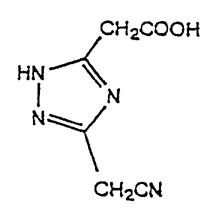 Melting point: 147 to 148 °C (decomposed)

## Claims

1. A compound; 3-(4-tert-butylphenyl)-1H-1,2,4-triazol-5-yl acetic acid. 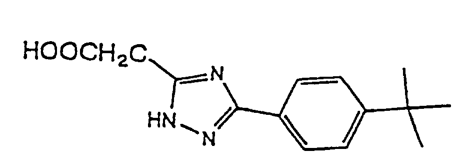 Use of the compound according to claim 1 for the production of photographic couplers.

## Patentansprüche

1. Verbindung: 3-(4-t-Butylphenyl)-1H-1,2,4-triazol-5-ylessigsäure:

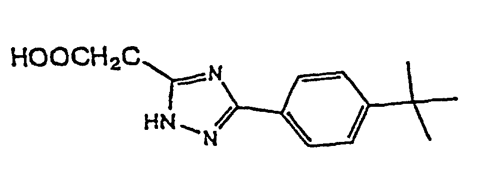

2. Verwendung der Verbindung gemäß Anspruch 1 zur Herstellung fotografischer Kuppler.

## Revendications

1. Composé; acide 3-(4-tert-butylphényl)-1H-1,2,4-triazol-5-yl-acétique.

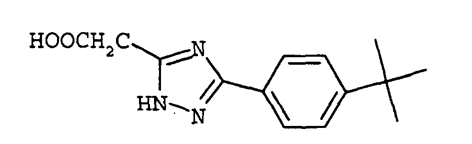

2. Utilisation du composé selon la revendication 1 pour la production de copulants en photographie.
